Europäisches Patentamt

European Patent Office

Office européen des brevets

(19)

(11) Veröffentlichungsnummer: **0 001 838**
**B1**

(12) **EUROPÄISCHE PATENTSCHRIFT**

(21) Anmeldenummer: **78101297.6**

(22) Anmeldetag: **03.11.78**

(51) Int. Cl.³: **C 07 G 7/00, C 12 N 7/02,**
**A 61 K 39/12, //**
**A 61 K 39/145**

(54) Verfahren zur Entfernung von Detergentien aus Virusantigensuspensionen

(30) Priorität: **09.11.77 DE 2750045**

(43) Veröffentlichungstag der Anmeldung:
**16.05.79 Patentblatt 79/10**

(45) Bekanntmachung des Hinweises auf die Patenterteilung:
**03.09.80 Patentblatt 80/18**

(84) Benannte Vertragsstaaten:
**BE CH DE FR GB LU NL SE**

(56) Entgegenhaltungen:
US - A - 3 790 552
Chemical Abstracts Band 67, Nr.9, 1967
Columbus, Ohio, USA
A. Caudwell "The use of high-molecular-weight,
water-soluble polymers for the extraction and
partial purification of plant viruses"
Seite 3824, Spalte 2, Abstract Nr. 40 849y
Chemical Abstracts Band 71, Nr. 13, 1969
Columbus, Ohio, USA
W. Matthaeus "Precipitation ion-exchange
chromatography, and electrophoretic mobility of
tissue-culture viruses in the presence of water-
soluble linear polycondensation and
polymerization products" Seite 148, Spalte 2,
Abstract Nr. 58 149n
Chemical Abstracts Band 75, Nr.4, 1971
Columbus Ohio, USA
L. C. Robinson et al "Purification, concentration,
and inactivation of vaccinia virus" Seite 312,
Spalte 2, Abstract Nr. 25 294 k
Chemical Abstracts Band 68, Nr.7, 1968
Columbus, Ohio, USA
J. C. Chermann et al "New method for purifying
endotoxins; precipitation by polyechylene glycol"
Seite 2706, Spalte 2 und Seite 2707, Spalte 1,
Abstract Nr. 28 087m

(73) Patentinhaber: **Behringwerke Aktiengesellschaft**
**Postfach 1140**
**D - 3550 Marburg**
**Lahn (DE)**

(72) Erfinder: **Reichert, Edgar, Dr.**
**Risberg 7**
**D - 3550 Marburg**
**Lahn (DE)**
**Majer, Mirko, Dr.**
**Lindenweg 11**
**D - 3550 Marburg**
**Lahn (DE)**

(74) Vertreter: **Meyer-Dulheuer, Karl-Hermann, Dr.**
**Hoechst Aktiengesellschaft Zentrale**
**Patentabteilung Postfach 80 03 20**
**D - 6230 Frankfurt**
**Main 80 (DE)**

Verfahren zur Entfernung von Detergentien aus Virusantigensuspensionen

Die Erfindung betrifft ein Verfahren zur Entfernung von Detergentien aus detergenzhaltigen Virusantigensuspensionen.

Das Influenzavirus ist kugelförmig. Seine Oberfläche ist mit stachelartigen Fortsätzen, den sogenannten "Spikes" besetzt, die dem Virus ein igelähnliches Aussehen verleihen. Es besitzt zwei verschiedene Typen von Spikes, das Hämagglutinin und die Neuraminidase.

Beide Spikesarten sind Glykoproteine. Sie allein sind für die immunisierenden Eigenschaften einer Influenzavaccine verantwortlich. Alle anderen Virusproteine sind für die Immunisierung ohne Bedeutung, sie können sogar durch Erhöhung des Proteingehaltes einer Vaccine deren Verträglichkeit negativ beeinflussen. Daher ist eine Influenza-Vaccine wünschenswert, die nur die beiden Oberflächenantigene enthält. Diese Oberflachenantigene lassen sich durch eine Anzahl von oberflachenaktiven Substanzen, sogenannten Detergentien, selektiv vom Virus abspalten, ohne daß der übrige Virus-Restkörper zerstört wird. Diese sonst unbeschädigten Virus-Restkörper lassen sich durch Ultrazentrifugation von den im Überstand verbleibenden Spikes abtrennen. Ein zu dieser selektien Spikesisolierung besonders gut geeignetes und bekanntes Detergens ist Triton-N-101 (Nonylphenoxypolyäthoxyäthanol).

Die durch Triton-N-101-Behandlung gewonnene, von Virus-Restkörpern freie Spikessuspension enthält auch das Triton-N-101. Sollen die auf diese Weise gewonnenen Spikes zur Herstellung einer Vaccine verwendet werden, ist es aus Verträglichkeits- und Stabilitätsgründen erforderlich, das Triton-N-101 zu entfernen.

Es sind schon Verfahren zur Entfernung des Detergens Triton-N-101 aus Virus-Spaltenansätzen beschrieben worden, die jedoch umständlich, verlustreich oder nicht ausreichend sind. So wurde eine Phasentrennung nach Erreichen des Trübungspunktes durch Erhöhung der Salzkonzentration vorgeschlagen. Da die Trennung jedoch nicht vollständig ist, muß ein weiterer Schritt angefügt werden, z.B. die Adsorption an Aluminiumhydroxid, die jedoch weiderum allein erst dann wirkungsvoll ist, wenn das Triton-N-101 in einer Mininmalkonzentration vorliegt. Nach der Adsorption an Aluminiumhydroxid lassen sich die Antigene anschließend nicht ohne größere Verluste von diesem eluieren. Auch eine Fällung mit Alkohol wurde schon beschrieben. Dabei besteht jedoch die Gefahr daß die Antigene denaturiert werden. Auch die Ultrafiltration ist schon genannt worden. Diese Methode kann jedoch nur bei Triton-Konzentrationen unter der kritischen Micellen-Konzentration von etwa 0,01 erfolgreich angewandt werden. Anderenfalls muß die Konzentration erniedrigt werden, z.B. durch Verdünnen, was wiederum die Ultrafiltration langwierig und verlustreich macht. Die Entfernung von Triton-N-101 mittels Adsorption an Bio Beads SM2 (Bio Rad Laboratories, USA), einem neutralen porösen Styrendivinylcopolymer in Perlenform, ist teuer. Die Vorbereitung der Bio Beads für die Adsorption ist umständlich und die Entfernung von Triton-N-101, das nicht in Micellenform vorliegt, ist nicht möglich. Daher kann durch Bio Beads die Tritonkonzentration nur bis auf eine Konzentration von etwa 0,01% entfernt werden, was nicht ausreichend ist.

Es wurde nun ein Verfahren zur Entfernung von Detergentien aus diese enthaltenden Virusantigensuspensionen gefunden, das dadurch gekennzeichnet ist, daß man die Virusantigene aus der detergenzhaltigen Virusantigensuspension mit Polyäthylenglykol ausfällt, das detergenzfreie Virusantigenpräzipitat durch Sedimentation gewinnt und gewünschtenfalls in einem Lösungsmittel aufnimmt.

Das Verfahren kann vorteilhaft ausgeführt werden, in dem man mit 6—18%, vorzugsweise 12%, Polyäthylenglykol ausfällt wobei das Molekulargewicht des verwendeten Polyäthylenglykols größer als 1000, vorzugsweise 6000, ist.

Das neue Verfahren kann auf alle Antigensuspensionen von Viren, die mit Detergentien gespalten werden können, angewandt werden. Dazu gehören die Influenzaviren, die Parainfluenzaviren und die Rhabdoviren, z.B. das Tollwutvirus. Als Detergens ist besonders das Nonylphenoxypolyäthoxyäthanol geeignet, das in der Literatur meist Triton-N-101 genannt wird. Triton ist die Bezeichnung für eine Reihe nichtionogener, oberflächenaktiver Stoffe. Man unterscheidet eine X- und eine N-Serie. Die Verbindungen der N-Serie, zu der Triton-N-101 gehört, entstehen durch Reaktion von Nonylphenol mit Äthylenoxid, Triton-N-101 ist ein Nonylphenoxypolyäthoxyäthanol, wobei die Silbe "poly" 9—10 Äthoxy-Gruppen bedeutet. Das Verfahren kann aber auch mit anderen Detergentien, z.B. mit Arkopal N 110 (Hoechst), ein nicht ionisches Detergens, mit Hoe S 2407 (Hoechst), ein anionisches Detergens, mit Genamin-O-080 (Hoechst), ein kationisches Detergens, und mit Hoe S 1982 (Hoechst), ein amphoterisches Detergens, ausgeführt werden.

Als Lösungsmittel im Sinne der Erfindung eignet sich Wasser sowie wäßrige Pufferlösungen im pH-Bereich in dem das jeweils verwendete Antigen am stabilisten ist. Beispielsweise liegt der günstigste pH-Wert zur Resuspendierung von Influenza-Oberflächenantigen bei etwa pH 7. Die Pufferlösungen können auch Neutralsalze enthalten. Bewährt hat sich z.B. Phosphat-gepufferte Kochsalzlösung.

Im Falle von Influenzaviren die mit etwa 1% des Detergens Triton-N-101 gespalten wurden, geht man zweckmäßigerweise so vor, daß man zunächst die vom unwirksamen Restpartikel abgespaltenen Oberflächenantigene durch Zentrifugation abtrennt. Dabei verbleibt das Detergens zusammen mit den Virusoberflächenantigenen in der überstehenden Suspension. Diese wird anschließend mit Polyäthylenglykol zweckmäßig mit einem solchen mit einem Molekulargewicht größer als 1000, vorzugsweise 6000, versetzt. Mit 12% Polyäthylenglykol wird dabei eine praktisch 100%ige Fällung erreicht. Die gefällten Antigene werden durch Zentrifugation isoliert und der detergenzahltige Überstand wird verworfen. Triton-N-101 kann nach diesem Fällungsschritt im Niederschlag nicht mehr nachgewiesen werden.

Gewünschtenfalls kann die Fällung der Virusantigene im gleichen Zentrifugenglas wiederholt werden, indem man das Sediment mit einer Pufferlösung auflöst und erneut mit Polyäthylenglykol ausfällt.

Das Sediment kann aber auch mit einer Pufferlösung, die mindestens 1% Polyäthylenglykol enthält, ausgewaschen werden. Löst man das Sediment in einer Pufferlösung, so ist die erhaltene Lösung detergenzfrei. Restliche Spuren von Polyäthylenglykol können gewünschtenfalls durch Ultrafiltration durch Membranen geeigneter Porenweite in bekannter Weise entfernt werden.

Triton-N-101 kann nach der von Gareval für Triton-N-101 beschriebenen Methode nachgewiesen werden. Die Methode beruht auf der Bildung eines blauen Präzipitates durch die Reaktion der Polyäthylenoxydgruppen des Triton mit Cobaltcyanat. Die blaue Farbe des Präzipitates wird durch Äthylendichlorid extrahiert und photometrisch ausgewertet.

Die erfindungsgemäß detergenzfrei gemachten Virusantigene können auf bekannte Weise zu Impfstoffen aufgearbeitet werden.

### Beispiel 1

Influenzavirus A Victoria wird auf bekannte Weise zwei Tage bei 35°C in embryonierten Hühnereiern vermehrt. Die virushaltigen Allantoisflüssigkeiten werden geerntet und gemischt. Das Virus wird durch Zuckergradientenzentrufigation in einer Elektronuecleionics-Ultrazentrifuge Modell K2 gereinigt und konzentriert.

Einem Liter des Viruskonzentrats werden 10 ml = 1% Triton-N-101 zugesetzt. Nach 3 Stunden Stehen bei 4°C werden die spikesfreien Viruskörper bei 53700 g 1 Stunde sedimentiert. Das Sediment wird verworfen. Dem Überstand werden 120 g = 12% Polyäthylenglykol 6000 zugesetzt. Nach 18 Stunden Inkubation bei 4°C werden die gefällten Spikes bei 10 000 UpM 30 Minuten sedimentiert. Das Sediment wird in phosphatgepufferter Kochsalzlösung aufgenommen. Es enthält die beiden Spikesarten des Virus, die Neuraminidase und das Hämagglutinin. Triton läßt sich in der Suspension nicht mehr nachweisen. Als Test dient die Methode von Gareval, deren Nachweisgrenze bei 0,005% Triton-N-101 liegt. Umgekehrt läßt sich im Überstand nach der Polyäthylenglykol-Fällung das eingesetzte Triton-N-101 quantitativ wiederfinden.

Das Hämagglutinin wird im Radical-Diffusions-Test nach Schild gegen Anti-Hämagglutinin-Serum identifiziert. Mittels Polyacrylamidgel-Elektrophorese werden drei Banden gefunden. Sie können den beiden Hämagglutininen und der Neuraminidase zugeordnet werden und sind typisch für die untersuchten Glykoproteine. Das auf diese Wiese isolierte Virusantigen wird in einer Menge von 1 ml unverdünnt und in den Verdünnungen $10^{-1}$ und $10^{-2}$ subcutan an je zwei Kaninchen verabreicht. Nach drei Wochen werden die Hämmagglutinationschemmungs - Antikörpertiter der Kaninchenseren gemessen. Sie betragen 128, 64, 16, d.h. in diesen Verdünnungen kommt es noch zu einer Antigen-Antikörper-Reaktion.

### Beispiel 2

Wie in Beispiel 1 beschrieben, wird Influenzavirus B Hongkong mit Polyäthylenglykol 6000 behandelt. Auch bei diesem Virus kann danach kein Triton-N-101 mehr nachgewiesen werden.

### Beispiel 3

Wie im Beispiel 1 beschrieben, wird Sendaivirus Stamm 52 mit Polyäthylenglykol 6000 behandelt. Auch bei diesem Virus kann danach im Sediment kein Triton-N-101 nachgewiesen werden.

### Beispiel 4

Tollwutvirus Stamm Pitman-Moore, vermehrt in menschlichen dipoiden Zellen Stamm WI-38 wird wie in Beispiel 1 beschrieben mit Triton-N-101 und anschließend mit Polyäthylenglykol behandelt. In der gewonnenen Antigensuspension läßt sich kein Triton nachweisen. Das Oberflächenantigen wird in der Elektrophorese als typische Bande nachgewiesen.

### Beispiel 5

Influenzavirus A Victoria wird durch 2% Triton über Nacht gespalten. Dabei werden nicht nur die Spikes abgelöst, sondern auch die Viruskörper aufgespalten. Durch Aufarbeitung der tritonhaltigen Suspension aller Virusantigene — wie in Beispiel 1 — erhält man ein Sediment, das alle Virusantigene enthält, aber frei von Triton ist.

### Beispiel 6

1 Liter eines — wie in Beispiel 1 gewonnenen-Viruskonzentrats des Influenzavirus A Victoria wird mit 10 g Arkopal N 110 behandelt. Nach Fällung mittels 12% Polyäthylenglykol kann in der Antigensuspension kein Arkopal N 110 nachgewiesen werden.

### Beispiel 7

Analog werden die Detergentien Hoe S

2407, Genamin O—080 und Hoe S 1982 eingesetzt. In allen Versuchen kann nach der Polyäthylenglykol-Fällung kein Detergens mehr nachgewiesen werden.

### Beispiel 8

Wie in Beispiel 1 beschrieben, werden die Spikes des Influenzavirus A Victoria

mit Polyäthylenglykol 1550,
mit Polyäthylenglykol 4000,
mit Polyäthylenglykol 20000 und
mit Polyäthylenglykol 40000

gefällt. In allen Versuchen enthält das Sediment die detergenzfreien Virusantigene.

### Beispiel 9

Wie im Beispiel 4 beschrieben, werden die mittels Triton-N-101 gewonnenen Antigene des Tollwutvirus Stamm Pitman-Moore.

mit Polyäthylenglykol 4000,
mit Polyäthylenglykol 20000 und
mit Polyäthylenglykol 40000

gefällt. In allen Versuchen enthält das Sediment die detergenzfreien Virusantigene.

**Patentansprüche**

1. Verfahren zur Entfernung von Detergentien aus diese enthaltenden Virusantigensuspensionen, dadurch gekennzeichnet, daß man die Virusantigene aus der detergenzhaltigen Virusantigensuspension mit Polyäthylenglykol ausfällt, das detergenzffreie Virusantigenpräzipitat durch Sedimentation gewinnt und gewünschtenfalls in einem Lösungsmittel aufnimmt.

2. Verfahren nach Anspruch 1, dadurch gekennzeichnet, daß als Lösungsmittel Wasser oder eine wäßrige Pufferlösung verwendet wird.

3. Verfahren nach Anspruch 1, dadurch gekennzeichnet, daß man mit 6—18%, vorzugsweise 12%, Polyäthylenglykol ausfällt.

4. Verfahren nach Anspruch 1, dadurch gekennzeichnet, daß das Molekulargewicht des verwendeten Polyäthylenglykols größer als 1000, vorzugsweise 6000, ist.

**Revendications**

1. Procédé pour éliminer les détergents de suspensions d'antigènes de virus contenant ces détergents, caractérisé en ce qu'on précipite les antigènes de virus de la suspension d'antigènes de virus contenant le détergent par du polyéthylène glycol, on recueille le produit précipité d'antigènes de virus sans détergent par sédimentation et on reprend éventuellement dans un solvant.

2. Procédé selon la revendication 1, caractérisé en ce que comme solvant on utilise de l'eau ou une solution tampon aqueuse.

3. Procéde selon la revendication 1, caractérisé en ce qu'on précipite avec de 6 à 18%, de préférence 12% de polyéthylène glycol.

4. Procédé selon la revendication 1, caractérisé en ce que le poids moléculaire du polyéthylène glycol utilisé est supérieur à 1000, de préférence égal à 6000.

**Claims**

1. A process for removing detergents from virus-antigen suspensions containing same, which comprises precipitating the virus-antigens from the detergent-containing virus-antigen suspension by adding polyethylene glycol and isolating the detergent-free virus-antigen precipitate by sedimentation and, optionally, taking up the precipitate in a liquid.

2. The process of claim 1, wherein the liquid medium is water or an aqueous buffer solution.

3. The process of claim 1, wherein the virus-antigens are precipitated by adding 6 to 18%, preferentially 12%, of polyethylene glycol.

4. The process of claim 1, wherein the molecular weight of the polyethylene glycol is above 1,000, preferentially 6,000.